# EUROPEAN PATENT SPECIFICATION

(11) **EP 4 511 069 B1**
(45) Date of publication and mention of the grant of the patent: **18.03.2026**
(21) Application number: 23724388.6
(22) Date of filing: 21.04.2023
(51) Int. Cl.: A61L 2/07, B01J 3/04

(54) **IMPROVED STERILISATION AUTOCLAVE**
PERFEKTIONIERTE STERILISATIONS-AUTOCLAVE
AUTOCLAVE DE STÉRILISATION PERFECTIONNÉE

(30) Priority: 22.04.2022 IT 202200007991
(43) Date of publication of application: 26.02.2025
(73) Proprietor: FEDEGARI AUTOCLAVI SPA, I-27010 Albuzzano (PV) (IT)
(72) Inventor: FEDEGARI, Paolo, 7505 CELERINA/SCHLARIGNA (CH); FEDEGARI, Giuseppe, 27100 PAVIA (IT)
(74) Representative: Grassi, Stefano
(86) International application number: PCT/IB2023/054093
(87) International publication number: WO 2023/203533

(56) References cited:
- DE-A1- 2 919 695
- FR-A- 940 097
- US-A- 2 713 702
- US-A1- 2010 143 218
- US-A1- 2012 039 766

## Description

### Technical field

The present invention relates to a sterilisation autoclave and method for sterilising objects and substances in the medical or chemical-pharmaceutical field.

In particular, the products to be sterilised are containers of various types, in glass, metals or other materials, indicatively for pharmaceutical, chemical and medical use.

### Prior art

With particular reference to the medical or chemical/pharmaceutical field, it is common practice to sterilise every medical device. This operation takes place with different methods according to the type of materials and devices to be sterilised and is codified by strict standards that aim at health protection and guarantee the safety of patients, hospital operators and the environment.

A commonly used sterilisation process envisages the exposure of the objects to substances to be sterilised at temperatures in the range between +110°C and +140°C through superheated steam, along with a possible baric treatment at pressures greater than or equal to ambient pressure (e.g. between -2 and 6 bar, preferably between 0 and 3 bar absolute), inside so-called stainless steel autoclaves.

To facilitate the loading and unloading operations of the objects or substances to be sterilised, stainless steel pallets are provided which allow the simultaneous stacking and treatment (insertion in the autoclave, sterilisation and removal from the autoclave) of a plurality of objects and substances subjected to the same sterilisation cycle.

To date, the mostly used process for the sterilisation of containers, for example in glass or metal, for pharmaceutical use consists of a sterilisation treatment with water vapour, for example, saturated and/or superheated at a constant predetermined temperature that lasts uninterruptedly for a predefined exposure time.

The sterilisation process with water vapour is very simple and extremely effective in terms of decontamination.

The typical operation cycle of the sterilisation autoclaves usually involves the following steps:
1. vacuum pump depressurisation;
2. one or more dynamic pulsations;
3. heating;
4. sterilisation;
5. cooling;
6. vacuum pump depressurisation.

Autoclaves of known type use steam spreading pipes for the introduction of steam inside the sterilisation chamber. The use of a modulating valve allows the steam inlet to be opened, closed and/or modulated during the various steps of operation of the sterilisation autoclave.

In the autoclaves of known type, moving components, such as fans, are used to carry out circulation of air and steam inside the sterilisation chamber.

Within the sterilisation autoclaves, more or less complex fluid-dynamic phenomena occur that involve physical mechanisms of different nature, which distinguish the specific process of the machine.

In particular, there are exchanges of matter such as condensation and evaporation of the fluid and thermal exchanges such as conduction, convection and heat radiation.

A problem with the autoclaves of known type lies in the slow diffusion and non-uniformity in the distribution of the water vapour within the sterilisation chamber.

Another problem with the autoclaves of known type is that the optimal temperature distribution for the sterilisation is initially slow and little uniform.

Another problem with the autoclaves of known type is the high energy consumption required for the operation of the autoclave during the steps described above.

A further problem of the autoclaves of known type lies in the possible formation of liquid film in the internal walls of the sterilisation chamber.

Another problem with the autoclaves of known type is the possible formation of water vapour stagnation zones.

A further problem of the autoclaves of known type lies in the possible formation of condensed water vapour at the products to be treated, which can even wet them.

Another problem with the autoclaves of known type is that they require the use of moving components to optimize the circulation of air and steam inside the sterilisation chamber.

A further problem of the autoclaves of known type lies in the fact that they require a certain interval of time to reach thermal uniformity over the entire internal volume of the autoclave.

Another problem with the autoclaves of known type lies in the fact that they do not allow to control the mass transfer phenomenon caused by the passage of state of the water vapour, in particular, to effectively control the field of motion and the actual amount of condensate.

Examples of devices operating in this technical field can be found in documents: US2010/143218A1, DE2919695A1, US2012/039766A1, US2713702A and FR940097A.

### Object of the invention

In this context, the object of the present invention is therefore to propose an improved sterilisation autoclave and sterilization method, according to claims 1 and 15 respectively, that allow to overcome the drawbacks indicated above with reference to the prior art.

Within this general object, a particular object of the present invention is to allow a rapid diffusion and uniformity in the distribution of the water vapour within the sterilisation chamber.

Another object of the present invention is to allow a rapid and uniform temperature distribution within the sterilisation chamber.

A further object of the present invention is to allow a low consumption of energy required for the operation of the autoclave during the steps described above.

Another object of the present invention is to prevent or minimize the formation of water vapour stagnation zones within the sterilisation chamber.

A further object of the present invention is to prevent or minimize the formation of condensed water vapour at the products to be treated.

Another object of the present invention is to carry out a circulation of air and steam inside the sterilisation chamber without the use of moving components.

A further object of the present invention is to optimize the process of circulation of air and steam inside the sterilisation chamber, also for the heating and cooling steps.

Another object of the present invention is to allow to have a thermal uniformity over the entire internal volume of the autoclave.

Not least object of the present invention is to allow to control the mass transfer phenomenon caused by the passage of state of the water vapour, in particular, to effectively control the field of motion and the effective amount of condensate.

### Brief description of the drawings

Additional features and advantages **will** become more apparent from the description of preferred but non-exclusive embodiments of a sterilisation or decontamination autoclave that are illustrated by way of indicative and non-limiting example in the appended drawings, in which:
- figure 1 shows a detail in perspective view of a sterilisation chamber with quadrangular section;
- figure 2 shows a front section of figure 1;
- figures 3 to 8 show a side section of a sterilisation chamber
- figure 9 shows an additional heating plate applicable to the sections from figure 3 up to figure 8;
- figure 10 shows a partial cross-section of a sterilization autoclave according to the invention;
- figure 11 shows a heating plate for the sterilisation chamber of figures 1 to 10;
- figure 11A shows a detail of the heating plate of figure 11.

### Detailed description of preferred embodiments of the invention

In a first aspect, the present invention describes a sterilisation autoclave or machine.

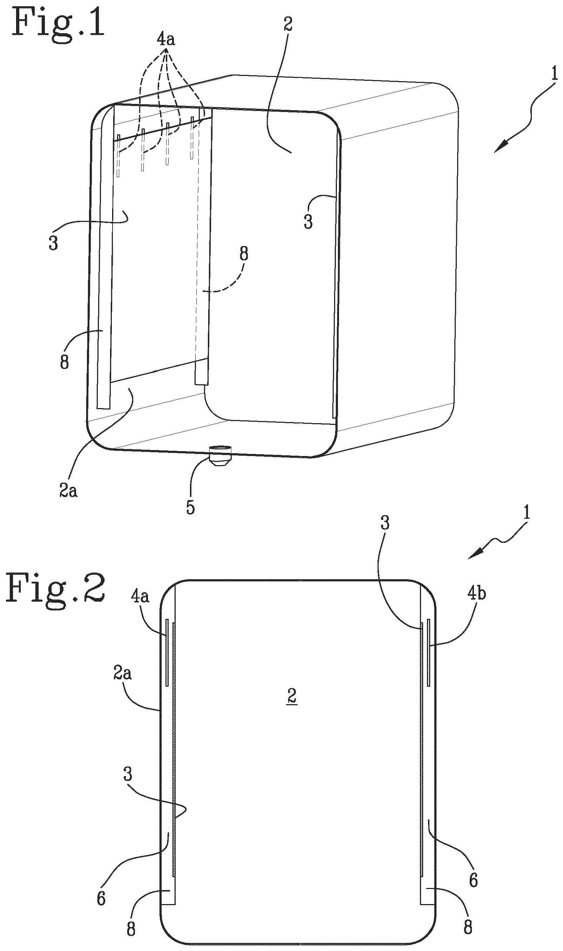

With reference to figure 1, a schematic view of a sterilisation or decontamination chamber 2 inside which the products to be sterilised are arranged is shown.

The sterilisation chamber 2 is contained within a sterilisation or decontamination autoclave 1.

The sterilisation chamber 2 is closed and is intended to contain the products to be sterilised in its inside, the chamber 2 identifies an internal compartment and is provided with an opening that identifies a passage for inserting/collecting the products to be sterilised.

A removable door associated when closed with said opening for passing from an open position, distanced from said opening, to a closed position in which it guarantees the hermetic closure of said passage of said sterilisation chamber 2.

In use, the chamber 2 is hermetically closed by the door and the sterilising agent (e.g., steam and/or sterilising gas) is fed therein as described below. The autoclave can have a cylindrical, quadrangular or any other shape.

The sterilisation chamber 2 can extend horizontally or vertically (like, for example, in the small mechanics used in laboratories).

In the present description, the vertical axis of the sterilisation machine 1 means an axis perpendicular to the floor on which the autoclave rests during its operation.

The sterilisation machine comprises at least one plate 3, for heating/cooling by means of process fluid, having two opposing sides with a greater extension and positioned in said sterilisation chamber 2 in proximity to a first wall 2a of said sterilisation chamber 2.

The plate is heated or cooled with a fluid, e.g., steam, air, or water.

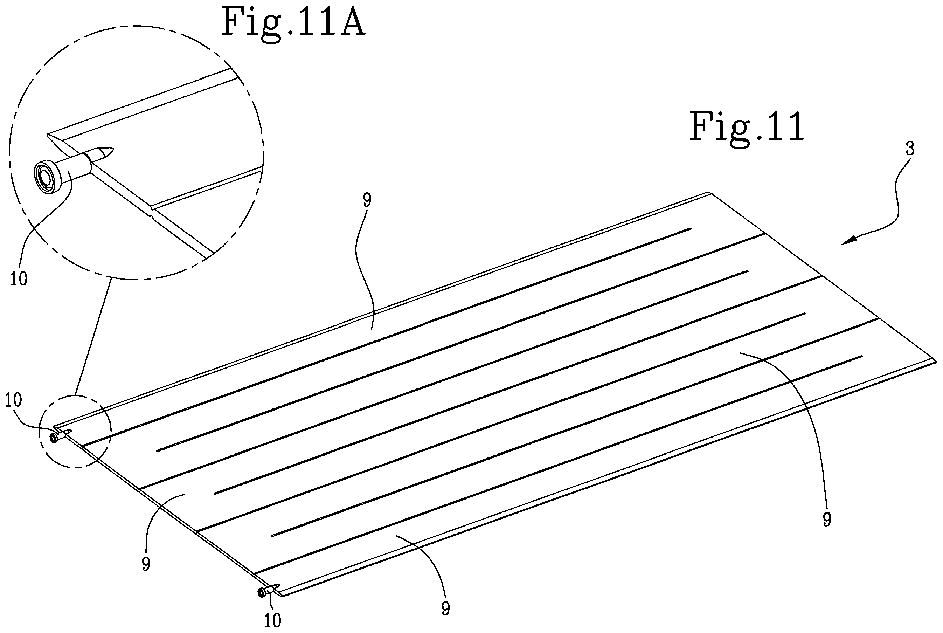

In accordance with the embodiment of figure 11, the heating plate 3 comprises a coil 9 which identifies a tubular passage for a working fluid (a liquid, a vapour or a gas), for heating or cooling.

Preferably, the heating plate 3 is shaped so as to identify the aforementioned coil 9 therein.

The coil 9 allows to ensure a heat exchange between the fluid that flows inside it and the sterilisation fluid that is present inside the sterilisation chamber and is in contact with the heating plate 3.

The geometry and the diameter of the coil 9 may vary depending on the specific technical requirements to be met.

As shown in the enlarged detail in figure 11A, the coil 9 comprises an inlet end 10 and an outlet end 10 positioned at opposing ends of the coil 9.

In accordance with one embodiment, the coil of the heating plate 3 consists of an electric coil. In this case, the heating plates 3 are heated by the electrical resistors forming the coil. In particular, in this case the heating means converts electrical energy into thermal energy which is irradiated inside the sterilisation chamber 2.

Optionally, it is possible to provide heating plates 3 comprising both the coil 9 with tubular passage for a fluid and the electric coil described above.

The plates 3, 3a, 3b, 3c, 3d, 3e, 3f, 3g have the following purposes:
1. To heat the mixed fluid present inside the gap 6;
2. To heat the internal wall 2a of the autoclave by irradiation;
3. To heat the rest of the fluid by convection and the load present inside chamber 2 by irradiation .

The plates 3 are also used in order to:
- pre-heat the load present inside the autoclave (in the first step, prior to sterilisation) in order to reduce condensates;
- dry the load once the sterilisation cycle is finished;
- cool the load prior to removal from the sterilisation chamber.

The plate 3 is positioned so as to have a first side of said two opposing sides facing said first wall 2a and comprises one or more nozzles 4a, 4b configured to introduce a flow of sterilising fluid, taken from the outside, into said sterilisation chamber 2.

The plate 3 identifies with the first wall an open gap 6 having a predefined separate secondary volume and in fluid communication with the remaining volume of the sterilisation chamber 2.

Preferably, the gap 6 is open at least at one or both of the lower and upper ends of each plate 3.

One or more nozzles 4a, 4b are positioned to introduce a flow of sterilising fluid directly into the open gap 6, so that the flow of sterilising fluid introduced into the gap 6 creates a suction dragging effect into said secondary volume of fluid present inside the remaining volume of the sterilisation chamber 2 ensuring fluid circulation in said sterilisation chamber 2.

The rapid introduction through the nozzles 4a, 4b of a hot flow of sterilising fluid, introduced into the gap 6, also entails the technical effect of heating, rapidly together with the plate 3, 3a-3g, one of the two opposing sides of the autoclave.

Heating the plate 3 allows heat to be radiated directly to the remaining volume of the sterilisation chamber (containing the products to be sterilised) and greatly reduces the amount of possible condensate that could form inside the chamber 2.

The sterilising fluid used may be air or steam or a mixture of air and steam, depending on the needs. The pressure upstream of the nozzle 4a, 4b will be that of the supply circuit of the machine (e.g. 6 barg for the air and 3.5 barg for the steam).

By exploiting the open gap 6 formed by the plates inside the chamber 2 and the position of the nozzles 4a, 4b to carry out circulation of the flow of fluid in the chamber, the Venturi effect in the channel 6 between the plates 3 and the internal part of the chamber 2 is intensified as much as possible. This phenomenon is similar to that of the mixing ejectors.

The ejectors are formed by two bodies, a nozzle 4a, 4b that is responsible for the introduction of the fluid at high flow rate and speed and by a Venturi-like body, the gap 6, which serves to mix the two flows and to suck the low pressure fluid present in the remaining volume.

The nozzle 4a, 4b of each ejector introduces steam into the chamber 2 while the external body will be obtained from the shape of the plate. Therefore, the assembly of nozzle 4a, 4b plus plates 3 is a single body to form the ejector.

The nozzles 4a, 4b of the ejectors can be connected at the inlet to the outlet of an external steam generator, to a steam supply system, to the compressed, filtered and conditioned air supply (e.g. with exchanger for the heating thereof).

Each ejector has a first inlet for a high pressure primary fluid (steam), a second inlet for a low pressure secondary fluid, a mixing chamber and an outlet, towards the inside of the sterilisation chamber 2, of the mixed primary and secondary fluids.

The diameter of the nozzles 4a, 4b is sized according to the flow rate and pressure conditions in the chamber that depend on the size of the machine and therefore of the chamber.

Preferably, the diameter of the inlet nozzle 4a, 4b is comprised between 10 mm and 20 mm, advantageously, it is equal to 15 mm.

The plates 3, 3a-3g, are heatable to a temperature comprised between 110°C and 140°C by circulating the flow of sterilising fluid within the gap 6 (e.g., steam).

Preferably, the plates 3 are heatable to a temperature of 400 Kelvin (126°C).

Advantageously, inside the sterilisation chamber 2 there are two plates 3 parallel to one another, positioned at a distance from respective opposite walls of the sterilisation chamber 2, each plate 3 defining an open gap 6, in which there are one or more nozzles 4a, 4b.

The plates 3 are preferably made of stainless steel and positioned on the opposite walls of the sterilisation chamber 2, parallel to the loading direction. To favour heat exchange phenomena, they can also extend on the upper part of the autoclave 1.

The shape is optimized to favour the flow of sterilising fluid inside the gap 6 that is formed between the internal wall of the autoclave and the plate 3 itself. The distance between plate 3 and the internal wall of the autoclave 1 is comprised between 10 and 100 mm and must be optimized according to the conditions of transport of the steam or of the air through nozzles 4a, 4b.

Preferably the distance between plate and internal wall of chamber 2 is between 30 mm and 50 mm

The heating fluid passing through each plate 3 can be steam or air with temperature approximately comprised between 110°C and 140°C depending on the sterilisation conditions and the step of the cycle. The plates 3 can also serve as a cooling system for the autoclave 1 and of the load.

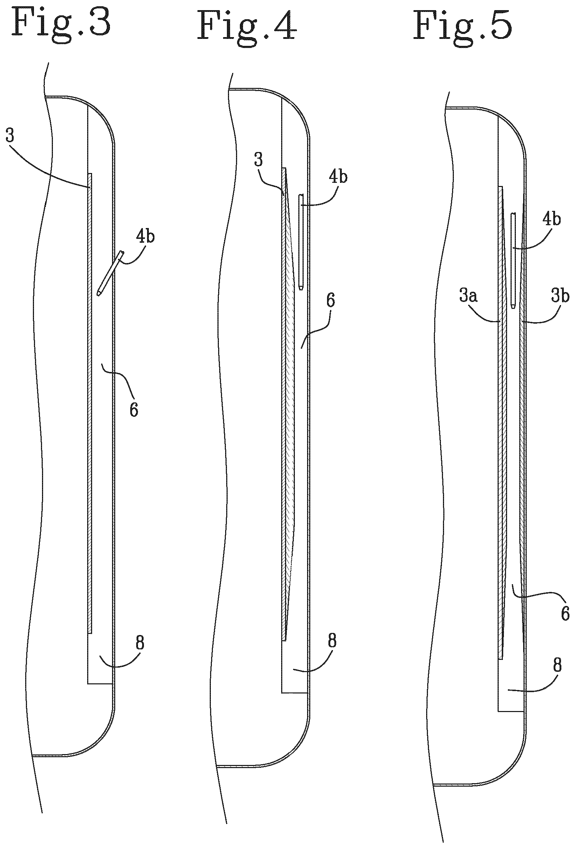

Various shapes and configurations of plates 3 are illustrated in figures 3 to 10.

A substantially flat plate 3 is illustrated in figure 3.

In figure 4 the plate is slightly concave on the side towards the inside of chamber 2 (convex towards a near wall of the chamber 2).

In figure 5, there are two plates 3a, 3b, a substantially flat plate 3b close to an internal wall of the chamber 2 and a plate 3a distanced from the plate 3b.

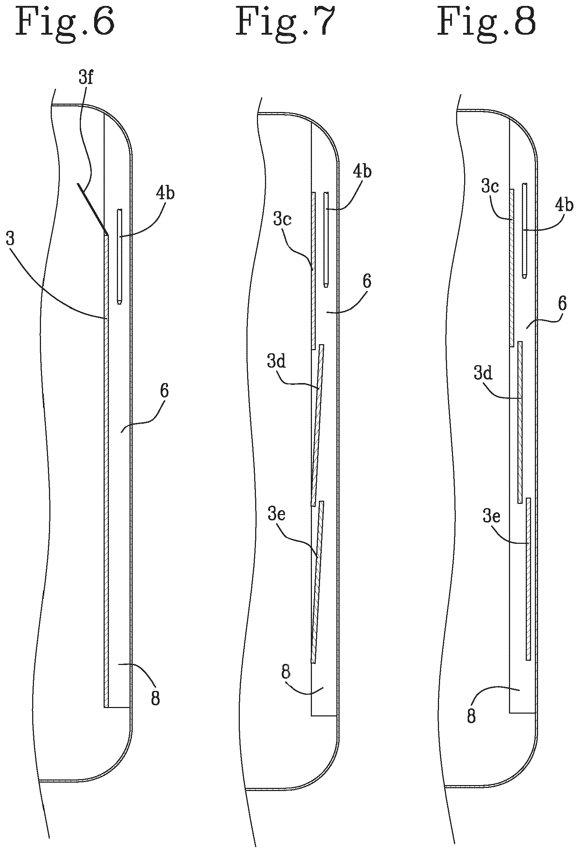

Figure 8 shows a plate 3c, 3d, 3e consisting of three flat elements, slightly shifted with respect to the vertical longitudinal axis, so as to define one or more passages for the flow of fluid between the secondary volume of the gap 6 and the remaining volume of the chamber 2.

Figure 7 shows a variant of the plate 3c, 3d, 3e of figure 8, in which at least two elements 3d and 3e are slightly inclined with respect to the vertical axis.

The plates (coils) arranged as shown in figures 7 or 8 also assume the function of baffles and allow a homogeneous distribution of the flow of sterilising gas (hot / cold steam or air) to different sterilisation chamber sections to optimize the heat exchange among the surfaces.

The combination of ejector and plates leads to an efficient distribution of the fluid (steam or air) inside the autoclave.

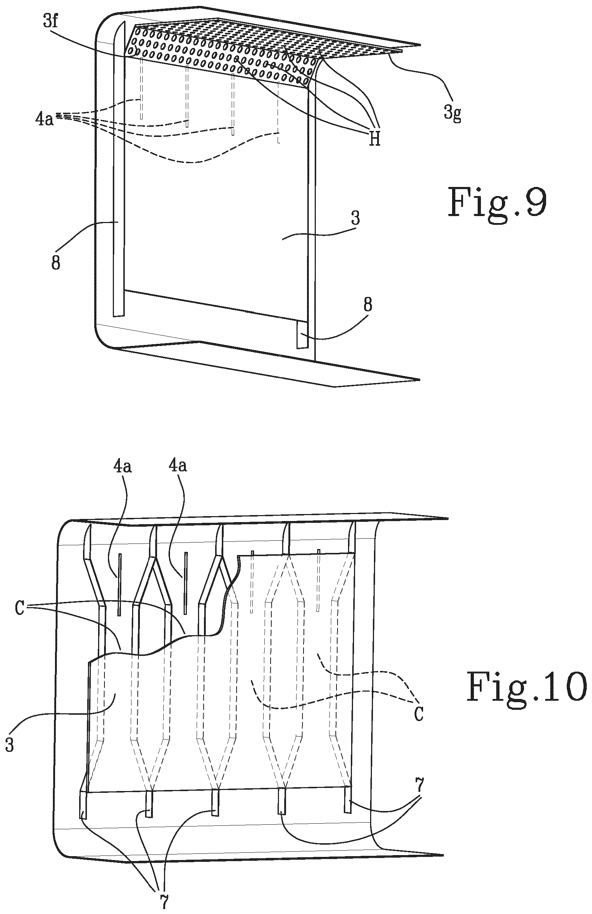

In figure 6 a variant is shown in which the upper open end of the plate 3 comprises a perforated deflecting baffle plate 3f, slightly inclined with respect to the vertical axis of the plate 3 (see also figure 9).

Figure 9 shows an embodiment in which the plate 3, 3b comprises at one end a perforated substantially horizontal plate 3g (i.e. perpendicular to the opposing sides of the plate 3).

The perforated horizontal plate 3g can be applied directly to the upper end of the vertical plate 3 or it can be applied as an appendage of the oblique perforated deflecting baffle plate 3f of figure 6.

In other words, figure 9 represents an additional heating plate 3g placed in the upper part of the chamber 2 and has the purpose of limiting any localized condensation phenomena.

Figure 9 can be applied to all sections starting from figure 3 up to figure 8.

The plates 3f and 3g may comprise a plurality of holes H configured to allow the passage of the sterilising fluid.

Figure 10 shows a gap 6 comprising one or more diversion elements 7 configured to convey the circulation of the flow of sterilising fluid along a plurality of channels C internal to the gap 6.

Each internal channel C is separated from one another and has a mouth with greater flow rate, at each nozzle 4a, 4b, a restricted central zone and a terminal zone with greater flow rate again, in such a way as to favour a sort of venturi effect to the flow of fluid and to optimize the effectiveness of the ejector.

There may be one or more lateral partitions 8, perpendicular to the plate 3, configured to laterally close the gap 6.

In a first embodiment, the nozzles 4a, 4b are with vertical axis, oriented vertically downwards of the sterilisation chamber 2 (figures 1, 2 and 4-10).

In this case, the plates 3 are advantageously distanced by 40 - 60 mm from the respective internal walls of the chamber 2.

In a second embodiment, the nozzles 4a, 4b have an inclination with respect to the vertical axis of the sterilisation chamber 2 comprised between 10° and 110°, preferably comprised between 40° and 80°.

The nozzles 4a, 4b can be positioned distanced from one another, at about half the height of each plate 3, i.e. the nozzles 4a, 4b are positioned half way along the distance between the lower part and the upper part of the plate 3.

In a constructional variant of the second embodiment, the nozzles 4a, 4b can be positioned distanced from one another, in proximity to the upper edge of the surface of each plate 3.

In the variants of the second embodiment, the plates 3 are advantageously distanced by 30 mm from the respective internal walls of the chamber 2.

In principle, each ejector will consist of an assembly of plate 3, 3a-3g, plus nozzle 4a, 4b.

The position of the nozzles 4a, 4b may vary in height from a minimum of a few tens of millimetres from the upper edge of the plate 3 to a maximum of about half height of the plate 3. As a result, the shapes of the plate and flow deflectors will change.

The inclination and the direction of the flow of the nozzles 4a, 4b will tend to be vertical and parallel to the plate and to the wall of the autoclave.

In one configuration the nozzle 4a, 4b is inclined by about 30° on the vertical and directed against the plate 3.

In the lower part of the sterilisation chamber, e.g. in the lower surface, parallel to the floor on which the sterilisation machine rests, there is a vent 5.

Preferably, the vent 5 is positioned, at the bottom, in a central position.

It is also possible to have an additional vent 5 positioned on the ceiling of the sterilisation chamber. In this case, since the sterilisation gas (e.g. steam) is lighter than the air, the emptying of the gas present inside the sterilisation chamber is accelerated. In this way the gas is sucked in (like in a hood) reducing the condensates present on the load. Any condensates that may form will be sucked with the vent 5 positioned at the bottom of the sterilisation chamber.

Preferably, the sterilising fluid is water vapour.

In a second aspect, the present invention describes a sterilisation method comprising the steps of:
- introducing the products to be sterilised into a sterilisation chamber (2) of a sterilisation autoclave (1) through a passage;
- hermetically closing the sterilisation chamber (2) with a movable door;
- introducing a flow of sterilising fluid directly into an open gap (6) present in the sterilisation chamber (2), delimited by a plate (3) distanced from one of the internal walls of the sterilisation chamber (2), by one or more nozzles (4a, 4b), so that said flow of sterilising fluid introduced into the gap (6) creates a suction dragging effect in said secondary volume of fluid present inside the remaining volume of the sterilisation chamber (2) ensuring fluid circulation in said sterilisation chamber (2). (2), said gap (6) comprising one or more diversion element (6) configured to convey the circulation of the flow of sterilising fluid along a plurality of channels (10) internal to the gap (6).

The present invention achieves the following technical effects:
- a faster and more uniform distribution of the temperature inside the sterilisation chamber 2;
- a faster and more uniform distribution of the sterilising fluid inside chamber 2;
- energy saving, with the same sterilising power of the machine;
- a decrease in the condensed mass of water;
- the plates 3 and the position of the injectors 4a, 4b allow carrying out a circulation of the steam inside the sterilisation chamber 2, intensifying as much as possible the venturi effect in the channel 6 between the plates 3 and the internal wall of the chamber;
- the presence of a channel between the plate and the internal wall is effective in carrying out an internal circulation, thanks to the positioning of the injectors, without the use of moving components;
- when the inlet and outlet flow rate is zero, a natural convective motion is triggered inside the chamber, generated by the thermal flow to the outside and by the plates kept at a constant temperature;
- even in these conditions the channel between the plate and the internal wall acts as a flow accelerator, exploiting the venturi effect, which could therefore be optimized even in a zero flow condition;
- maintaining the plate at a high temperature allows local heating of the steam tending to rise in the part of the chamber inside the plates;
- the amount of liquid (condensed steam) is very low and tends to accumulate in the lower part of the autoclave.

The invention as it is conceived is susceptible to numerous modifications and variants, all falling within the scope of the claims.

Furthermore, all of the details can be replaced with other technically equivalent elements. In practice, all the materials used, as well as the dimensions, can be any whatsoever, according to need.

## Claims

1. A sterilisation autoclave (1) comprising:
- a closed sterilisation chamber (2) intended to contain the products to be sterilised, said chamber (2) identifying an internal compartment and being provided with an opening that identifies a passage for inserting/collecting the products to be sterilised;
- a removable door associated when closed with said opening for passing from an open position, distanced from said opening, to a closed position in which it guarantees the hermetic closure of said passage of said sterilisation chamber (2);
- a plate (3) having two opposing sides with a greater extension and positioned in said sterilisation chamber (2) in proximity to a first wall (2a) of said sterilisation chamber (2), said plate (3) being positioned so as to have a first side of said two opposing sides facing said first wall (2a) and
- one or more nozzles (4a, 4b) configured to introduce a flow of sterilising fluid into said sterilisation chamber (2),
wherein:
- said plate (3) identifies with said first wall (2a) an open gap (6) having a predefined separate secondary volume and in fluid communication with the remaining volume of the sterilisation chamber (2),
- said one or more nozzles (4a, 4b) are positioned to introduce a flow of sterilising fluid directly into said open gap (6), so that said flow of sterilising fluid introduced into the gap (6) creates a suction dragging effect into said secondary volume of fluid present inside the remaining volume of the sterilisation chamber (2) ensuring fluid circulation in said sterilisation chamber (2),
and **characterised by**,
- one or more diversion elements (7) positioned inside the gap (6), said one or more diversion elements (7) being configured to convey the circulation of the flow of sterilising fluid along a plurality of channels (C) internal to the gap (6).

2. The autoclave according to claim 1, wherein inside the sterilisation chamber (2) there are two plates (3) parallel to one another, positioned at a distance from respective opposite walls of the sterilisation chamber (2), each plate (3) defining an open gap (6), in which there are one or more nozzles (4a, 4b).

3. The autoclave according to claim 2, wherein each plate (3) is distanced from an internal wall of the sterilisation chamber (2) between 10 mm and 100 mm, preferably between 30 mm and 50 mm.

4. The autoclave according to one or more of the preceding claims, wherein at least one plate (3) is:
- flat;
- convergent/divergent;
- composed of two flat plates (3a, 3b), a first plate (3a) positioned close to the internal wall of the chamber (2) and a second plate (3b) distanced from said first plate (3a);
- composed of a plurality of flat elements (3c, 3b, 3e) positioned so as to define one or more passages for the flow of fluid between the secondary volume of the gap (6) and the remaining volume of the chamber (2).

5. The autoclave according to one or more of the preceding claims, wherein said plate (3, 3b) comprises at one end a perforated deflecting baffle plate (3f).

6. The autoclave according to one or more of the preceding claims, wherein said plate (3, 3b) comprises at one end a perforated horizontal plate (3g).

7. The autoclave according to one or more of the preceding claims, where there are one or more lateral partitions (8), perpendicular to said plate (3), configured to laterally close the gap (6).

8. The autoclave according to any of the preceding claims, wherein the longitudinal axis of the nozzles (4a, 4b) is vertical.

9. The autoclave according to any of the preceding claims 1 to 7, wherein the longitudinal axis of the nozzles (4a, 4b) has an inclination with respect to the vertical axis of the sterilisation chamber (2) comprised between 10° and 110°, preferably comprised between 40° and 80°.

10. The autoclave according to any of the preceding claims, wherein the nozzles (4a, 4b) are positioned half way along the distance between a lower part and an upper part of each plate (3).

11. The autoclave according to any of the preceding claims 1 to 9, wherein the nozzles (4a, 4b) are positioned in the upper part of the plate (3).

12. The autoclave according to any of the preceding claims, wherein the at least one plate (3) identifies a coil (9) consisting of a tubular passage to let a working fluid flow therein, said coil (9) comprising an inlet end (10) and an outlet end (10) of the fluid.

13. The autoclave according to any of the preceding claims 1 to 11, wherein the at least one plate (3) comprises an electrical coil configured to convert electrical energy into thermal energy.

14. The autoclave according to any of the preceding claims wherein said plate (3, 3a, 3b, 3c, 3d, 3e, 3f, 3g) is heated/cooled by a process fluid or other system.

15. A sterilisation method comprising the steps of:
- introducing the products to be sterilised into a sterilisation chamber (2) of a sterilisation autoclave (1) through a passage;
- hermetically closing the sterilisation chamber (2) with a movable door;
- introducing a flow of sterilising fluid directly into an open gap (6) present in the sterilisation chamber (2), delimited by a plate (3) distanced from one of the internal walls of the sterilisation chamber (2), by one or more nozzles (4a, 4b), so that said flow of sterilising fluid introduced into the gap (6) creates a suction dragging effect in said secondary volume of fluid present inside the remaining volume of the sterilisation chamber (2) ensuring fluid circulation in said sterilisation chamber (2);
**characterised by**,
said
gap (6) comprising one or more diversion element (6) configured to convey the circulation of the flow of sterilising fluid along a plurality of channels (10) internal to the gap (6).

## Patentansprüche

1. Sterilisationsautoklav (1), umfassend:
- eine geschlossene Sterilisationskammer (2), die dazu bestimmt ist, die zu sterilisierenden Produkte aufzunehmen, wobei die Kammer (2) einen Innenraum identifiziert und mit einer Öffnung versehen ist, die einen Durchgang zum Einführen/Sammeln der zu sterilisierenden Produkte identifiziert;
- eine abnehmbare Tür, die im geschlossenen Zustand mit der Öffnung assoziiert ist, um von einer offenen Position, die von der Öffnung beabstandet ist, in eine geschlossene Position zu gelangen, in der sie den hermetischen Verschluss des Durchgangs der Sterilisationskammer (2) garantiert;
- eine Platte (3) mit zwei entgegengesetzten Seiten mit größerer Ausdehnung, die in der Sterilisationskammer (2) in der Nähe einer ersten Wand (2a) der Sterilisationskammer (2) positioniert ist, wobei die Platte (3) so positioniert ist, dass eine erste Seite der zwei entgegengesetzten Seiten der ersten Wand (2a) zugewandt ist, und
- eine oder mehrere Düsen (4a, 4b), die ausgelegt sind, um einen Strom von Sterilisationsfluid in die Sterilisationskammer (2) einzuführen,
wobei:
- die Platte (3) mit der ersten Wand (2a) einen offenen Spalt (6) identifiziert, der ein vordefiniertes separates Sekundärvolumen aufweist und in Fluidkommunikation mit dem verbleibenden Volumen der Sterilisationskammer (2) steht,
- die eine oder mehreren Düsen (4a, 4b) so positioniert sind, dass sie einen Strom von Sterilisationsfluid direkt in den offenen Spalt (6) einführen, so dass der Strom von Sterilisationsfluid, der in den Spalt (6) eingeführt wird, eine Saugschleppwirkung in das sekundäre Fluidvolumen erzeugt, das innerhalb des verbleibenden Volumens der Sterilisationskammer (2) vorhanden ist, wodurch eine Fluidzirkulation in der Sterilisationskammer (2) gewährleistet wird,
und **gekennzeichnet durch**
- ein oder mehrere Umlenkelemente (7), die innerhalb des Spalts (6) positioniert sind, wobei das eine oder die mehreren Umlenkelemente (7) dazu ausgelegt sind, die Zirkulation des Stroms von Sterilisationsfluid entlang einer Vielzahl von Kanälen (C) innerhalb des Spalts (6) zu befördern.

2. Autoklav nach Anspruch 1, wobei innerhalb der Sterilisationskammer (2) zwei Platten (3) parallel zueinander vorhanden sind, die in einem Abstand von jeweils gegenüberliegenden Wänden der Sterilisationskammer (2) positioniert sind, wobei eine jede Platte (3) einen offenen Spalt (6) definiert, in dem sich eine oder mehrere Düsen (4a, 4b) befinden.

3. Autoklav nach Anspruch 2, wobei eine jede Platte (3) von einer Innenwand der Sterilisationskammer (2) zwischen 10 mm und 100 mm, vorzugsweise zwischen 30 mm und 50 mm, beabstandet ist.

4. Autoklav nach einem oder mehreren der vorhergehenden Ansprüche, wobei mindestens eine Platte (3):
- flach ist;
- konvergent/divergent ist;
- aus zwei flachen Platten (3a, 3b) besteht, wobei eine erste Platte (3a) nahe der Innenwand der Kammer (2) positioniert ist und eine zweite Platte (3b) von der ersten Platte (3a) beabstandet ist;
- aus einer Vielzahl von flachen Elementen (3c, 3b, 3e) besteht, die so positioniert sind, dass sie einen oder mehrere Durchgänge für den Fluidstrom zwischen dem Sekundärvolumen des Spalts (6) und dem verbleibenden Volumen der Kammer (2) definieren.

5. Autoklav nach einem oder mehreren der vorhergehenden Ansprüche, wobei die Platte (3, 3b) an einem Ende eine perforierte ablenkende Ablenkplatte (3f) umfasst.

6. Autoklav nach einem oder mehreren der vorhergehenden Ansprüche, wobei die Platte (3, 3b) an einem Ende eine perforierte horizontale Platte (3g) umfasst.

7. Autoklav nach einem oder mehreren der vorhergehenden Ansprüche, wobei eine oder mehrere seitliche Trennwände (8) senkrecht zu der Platte (3) vorhanden sind, die ausgelegt sind, um den Spalt (6) seitlich zu schließen.

8. Autoklav nach einem der vorhergehenden Ansprüche, wobei die Längsachse der Düsen (4a, 4b) vertikal ist.

9. Autoklav nach einem der vorhergehenden Ansprüche 1 bis 7, wobei die Längsachse der Düsen (4a, 4b) eine Neigung in Bezug auf die vertikale Achse der Sterilisationskammer (2) zwischen 10° und 110°, vorzugsweise zwischen 40° und 80° aufweist.

10. Autoklav nach einem der vorhergehenden Ansprüche, wobei die Düsen (4a, 4b) auf halber Strecke zwischen einem unteren Teil und einem oberen Teil einer jeden Platte (3) positioniert sind.

11. Autoklav nach einem der vorhergehenden Ansprüche 1 bis 9, wobei die Düsen (4a, 4b) im oberen Teil der Platte (3) positioniert sind.

12. Autoklav nach einem der vorhergehenden Ansprüche, wobei die mindestens eine Platte (3) eine Spule (9) identifiziert, die aus einem rohrförmigen Durchgang besteht, um ein Arbeitsfluid darin strömen zu lassen, wobei die Spule (9) ein Einlassende (10) und ein Auslassende (10) des Fluids umfasst.

13. Autoklav nach einem der vorhergehenden Ansprüche 1 bis 11, wobei die mindestens eine Platte (3) eine elektrische Spule umfasst, die dazu ausgelegt ist, elektrische Energie in thermische Energie umzuwandeln.

14. Autoklav nach einem der vorhergehenden Ansprüche, wobei die Platte (3, 3a, 3b, 3c, 3d, 3e, 3f, 3g) durch ein Prozessfluid oder ein anderes System erwärmt/gekühlt wird.

15. Sterilisationsverfahren, umfassend die folgenden Schritte:
- Einführen der zu sterilisierenden Produkte in eine Sterilisationskammer (2) eines Sterilisationsautoklavs (1) durch einen Durchgang;
- hermetisches Verschließen der Sterilisationskammer (2) mit einer beweglichen Tür;
- Einführen eines Stroms von Sterilisationsfluid direkt in einen offenen Spalt (6), der in der Sterilisationskammer (2) vorhanden ist, der durch eine Platte (3) begrenzt ist, die von einer der Innenwände der Sterilisationskammer (2) durch eine oder mehrere Düsen (4a, 4b) beabstandet ist, so dass der Strom von Sterilisationsfluid, der in den Spalt (6) eingeführt wird, eine Saugschleppwirkung in das sekundäre Fluidvolumen erzeugt, das in dem verbleibenden Volumen der Sterilisationskammer (2) vorhanden ist, wodurch die Fluidzirkulation in der Sterilisationskammer (2) gewährleistet wird;
**dadurch gekennzeichnet, dass**
der Spalt (6) ein oder mehrere Umlenkelemente (6) umfasst, die dazu ausgelegt sind, die Zirkulation des Stroms von Sterilisationsfluid entlang einer Vielzahl von Kanälen (10) innerhalb des Spalts (6) zu befördern.

## Revendications

1. Autoclave de stérilisation (1), comprenant :
- une chambre de stérilisation (2) fermée destinée à contenir les produits à stériliser, ladite chambre (2) identifiant un compartiment interne et étant pourvue d'une ouverture qui identifie un passage pour l'insertion/la collecte des produits à stériliser ;
- une porte amovible associée, lorsqu'elle est fermée, à ladite ouverture pour passer d'une position ouverte, éloignée de ladite ouverture, à une position fermée dans laquelle elle garantit la fermeture hermétique dudit passage de ladite chambre de stérilisation (2) ;
- une plaque (3) présentant deux côtés opposés de plus grande extension et positionnée dans ladite chambre de stérilisation (2) à proximité d'une première paroi (2a) de ladite chambre de stérilisation (2), ladite plaque (3) étant positionnée de manière à avoir un premier côté desdits deux côtés opposés faisant face à ladite première paroi (2a) et
- une ou plusieurs buses (4a, 4b) configurées pour introduire un flux de fluide de stérilisation dans ladite chambre de stérilisation (2),
dans lequel :
- ladite plaque (3) identifie avec ladite première paroi (2a) un espace ouvert (6) ayant un volume secondaire séparé prédéfini et en communication fluidique avec le volume restant de la chambre de stérilisation (2),
- ladite ou lesdites buses (4a, 4b) est/sont positionnée(s) pour introduire un flux de fluide de stérilisation directement dans ledit espace ouvert (6), de sorte que ledit flux de fluide de stérilisation introduit dans l'espace (6) crée un effet d'entraînement par aspiration dans ledit volume secondaire de fluide présent à l'intérieur du volume restant de la chambre de stérilisation (2) assurant la circulation du fluide dans ladite chambre de stérilisation (2),
et **caractérisé en ce que**
- un ou plusieurs éléments de déviation (7) positionnés à l'intérieur de l'espace (6), ledit ou lesdits éléments de déviation (7) étant configuré(s) pour acheminer la circulation du flux de fluide de stérilisation le long d'une pluralité de canaux (C) internes à l'espace (6).

2. Autoclave selon la revendication 1, dans lequel à l'intérieur de la chambre de stérilisation (2) se trouvent deux plaques (3) parallèles l'une à l'autre, positionnées à une certaine distance des parois opposées respectives de la chambre de stérilisation (2), chaque plaque (3) définissant un espace ouvert (6), dans lequel se trouvent une ou plusieurs buses (4a, 4b).

3. Autoclave selon la revendication 2, dans lequel chaque plaque (3) est espacée d'une paroi interne de la chambre de stérilisation (2) d'une distance comprise entre 10 et 100 mm, de préférence entre 30 et 50 mm.

4. Autoclave selon l'une ou plusieurs des revendications précédentes, dans lequel au moins une plaque (3) est :
- plate ;
- convergente/divergente ;
- composée de deux plaques plates (3a, 3b), une première plaque (3a) positionnée près de la paroi interne de la chambre (2) et une seconde plaque (3b) espacée de ladite première plaque (3a) ;
- composée d'une pluralité d'éléments plats (3c, 3b, 3e) positionnés de manière à définir un ou plusieurs passages pour l'écoulement du fluide entre le volume secondaire de l'espace (6) et le volume restant de la chambre (2).

5. Autoclave selon l'une ou plusieurs des revendications précédentes, dans lequel ladite plaque (3, 3b) comprend à une extrémité une chicane de déflection perforée (3f).

6. Autoclave selon l'une ou plusieurs des revendications précédentes, dans lequel ladite plaque (3, 3b) comprend à une extrémité une plaque horizontale perforée (3g).

7. Autoclave selon l'une ou plusieurs des revendications précédentes, où se trouvent une ou plusieurs cloisons latérales (8), perpendiculaires à ladite plaque (3), configurées pour fermer latéralement l'espace (6).

8. Autoclave selon l'une quelconque des revendications précédentes, dans lequel l'axe longitudinal des buses (4a, 4b) est vertical.

9. Autoclave selon l'une quelconque des revendications précédentes 1 à 7, dans lequel l'axe longitudinal des buses (4a, 4b) présente une inclinaison par rapport à l'axe vertical de la chambre de stérilisation (2) comprise entre 10 et 110°, de préférence comprise entre 40 et 80°.

10. Autoclave selon l'une quelconque des revendications précédentes, dans lequel les buses (4a, 4b) sont positionnées à mi-chemin le long de la distance entre une partie inférieure et une partie supérieure de chaque plaque (3).

11. Autoclave selon l'une quelconque des revendications précédentes 1 à 9, dans lequel les buses (4a, 4b) sont positionnées dans la partie supérieure de la plaque (3).

12. Autoclave selon l'une quelconque des revendications précédentes, dans lequel l'au moins une plaque (3) identifie un serpentin (9) constitué d'un passage tubulaire pour laisser s'écouler un fluide de travail à l'intérieur, ledit serpentin (9) comprenant une extrémité d'entrée (10) et une extrémité de sortie (10) du fluide.

13. Autoclave selon l'une quelconque des revendications précédentes 1 à 11, dans lequel l'au moins une plaque (3) comprend une bobine électrique configurée pour convertir l'énergie électrique en énergie thermique.

14. Autoclave selon l'une quelconque des revendications précédentes, dans lequel ladite plaque (3, 3a, 3b, 3c, 3d, 3e, 3f, 3g) est chauffée/refroidie par un fluide de traitement ou un autre système.

15. Procédé de stérilisation comprenant les étapes suivantes :
- introduire les produits à stériliser dans une chambre de stérilisation (2) d'un autoclave de stérilisation (1) à travers un passage ;
- fermer hermétiquement la chambre de stérilisation (2) à l'aide d'une porte mobile ;
- introduire un flux de fluide de stérilisation directement dans un espace ouvert (6) présent dans la chambre de stérilisation (2), délimité par une plaque (3) espacée de l'une des parois internes de la chambre de stérilisation (2), par une ou plusieurs buses (4a, 4b), de sorte que ledit flux de fluide de stérilisation introduit dans l'espace (6) crée un effet d'entraînement par aspiration dans ledit volume secondaire de fluide présent à l'intérieur du volume restant de la chambre de stérilisation (2) assurant la circulation du fluide dans ladite chambre de stérilisation (2) ;
**caractérisé en ce que**
ledit espace (6) comprend un ou plusieurs éléments de déviation (6) configurés pour acheminer la circulation du flux de fluide de stérilisation le long d'une pluralité de canaux (10) internes à l'espace (6).
